# EUROPEAN PATENT SPECIFICATION

(11) **EP 1 558 652 B1**
(45) Date of publication and mention of the grant of the patent: **11.02.2009**
(21) Application number: 03778489.9
(22) Date of filing: 05.11.2003
(51) Int. Cl.: C08F 8/00

(54) **DERIVATISED HYDROGELS AND THEIR USE**
MODIFIZIERTE HYDROGELE UND IHRE VERWENDUNG
HYDROGELS DERIVES ET LEUR UTILISATION

(30) Priority: 05.11.2002 GB 0225761
(43) Date of publication of application: 03.08.2005
(73) Proprietor: AGT Sciences Limited, Listerhills Science Park Bradford West Yorkshire BD7 1HR (GB)
(72) Inventor: BRITLAND, Stephen T., Bradford, West Yorkshire BD7 1DP (GB); CROWTHER, Nicholas John, Bradford, West Yokshire BD13 4LU (GB); EAGLAND, Donald, Huddersfield, West Yorkshire HD8 0JJ (GB)
(74) Representative: Brierley, Anthony Paul
(86) International application number: PCT/GB2003/004791
(87) International publication number: WO 2004/041872

(56) References cited:
- EP-A- 0 227 526
- WO-A-01/70285
- FR-A- 2 790 391

## Description

This invention relates to polymeric materials and particularly, although not exclusively, relates to such materials in the form of hydrogels. Preferred embodiments relate to the use of a polymeric material in a biological application, such as in tissue engineering, for example to support cell amplification; as a vehicle for cell or tissue grafting or transplantation; and/or as a basis for "smart" wound dressings.

Several types of traumatic injury and several disease states can affect quality of life by prejudicing normal functioning of different organ systems. Examples include lesions to the cornea of the eye, impact and abrasive damage to the surface of articular cartilage, and acute trauma or chronic wounds affecting the skin. In the absence of sufficient or appropriate intact tissues for transplantation or grafting to treat such conditions, one potential therapeutic strategy would be to take a small biopsy from the patient and increase the numbers of cells in vitro using culture techniques to produce autologous tissues for use in treating the same patient. Certain cells when grown in culture will in any case re-establish simple tissue architectures such as epithelium given appropriate conditions. Cell cultures can be harvested and deployed from such tissue engineered epithelial sheets in several different ways. For example, the cells may be transferred to another surface to which they adhere and can continue to survive. One object of the invention is to provide a polymeric material that can support cell growth/amplification.

So called "smart" dressings are known which are adapted to facilitate the two cellular processes which are pivotal to successful wound repair, namely cell division and cell migration. Both of the processes are substratum dependent. Pathological conditions that impair or impede either of the processes can lead to the formation of chronic, poorly healing wounds and possibly significant scarring. Another object of the present invention is to provide a polymeric material that can be used in a "smart" dressing.

Polymeric materials in the form of hydrogels are known for a range of uses. WO98/12239 (University of Bradford) describes a range of such hydrogels. Hydrogels comprising polyvinylalcohol cross-linked by glutaraldelyde are also known. However, such known hydrogels are not in themselves generally suitable for use in cell amplification or smart dressings. It is one object of the present invention to provide a hydrogel which has a wide range of useful applications.

According to a first aspect of the invention there is provided a method of derivatising a polymeric material of a type which includes encapsulated water, the method comprising:
(a) selecting a first hydrated polymeric material which includes encapsulated water;
(b) reducing the level of encapsulated water in said first hydrated polymeric material to produce a second polymeric material;
(c) treating said second polymeric material with derivatisation means for derivatising said second polymeric material.

Said first hydrated polymeric material is preferably a hydrogel. A said hydrogel may be defined as a cross-linked, water insoluble, water containing material.

Said first polymeric material or hydrogel suitably contains at least 40wt%, preferably at least 55wt%, more preferably at least 70wt%, especially at least 80wt% water. The amount of water may be less than 95wt%, preferably less than 90wt%. The level of water may be determined by any suitable means, for example by thermogravimetric analysis.

The difference between the wt% of water in said first polymeric material and that in said second polymeric material may be at least 40wt%, preferably at least 55wt%, more preferably at least 70wt%. The ratio of the amount (wt%) of water in the first polymeric material to that in said second polymeric material may be at least 10, suitably at least 20, preferably at least 30, more preferably at least 40, especially at least 50. The ratio is preferably less than 1000.

Said second polymeric material suitably includes less than 10wt%, preferably less than 5wt%, more preferably less than 2wt%, especially less than 1wt% of encapsulated water. Said second polymeric material may include a trace, for example at least 0.01wt% of encapsulated water.

Said first hydrated polymeric material preferably comprises a third polymeric material which is cross-linked by a cross-linking means. Said first polymeric material may be prepared by selecting a third polymeric material and treating it with a said cross-linking means. Wherein said third polymeric material may include functional groups selected from hydroxy, carboxylic acid, carboxylic acid derivatives (e.g. ester) and amine groups. Said third polymeric material preferably includes a backbone comprising, preferably consisting essentially of carbon atoms. The backbone is preferably saturated. Pendent from the backbone are one or more said functional groups described. Said third polymeric material may have a molecular weight of at least 10,000. Said third polymeric material is preferably a polyvinyl polymer. Preferred third polymeric materials include optionally substituted, preferably unsubstituted, polyvinylalcohol, polyvinylacetate, polyalkylene glycols, for example polypropylene glycol, and collagen (and any component thereof). Polyvinylalcohol is an especially preferred third polymeric material.

In especially preferred embodiments said first polymeric material include cross-linked polyvinyl alcohol.

A preferred cross-linking means comprises a chemical cross-linking material. Such a material is preferably a polyfunctional compound having at least two functional groups capable of reacting with functional groups of said third polymeric material. Preferably, said cross-linking material includes one or more of carbonyl, carboxyl, hydroxy, epoxy, halogen or amino functional groups which are capable of reacting with groups present along the polymer backbone or in the polymer structure of the third polymeric material. Preferred cross-linking materials include at least two aldehyde groups. Thus, in a preferred embodiment, said first polymeric material includes a material formed by cross-linking polyvinylalcohol using a material having at least two aldehyde groups. Thus, said first polymeric material preferably includes a moiety of formula I. 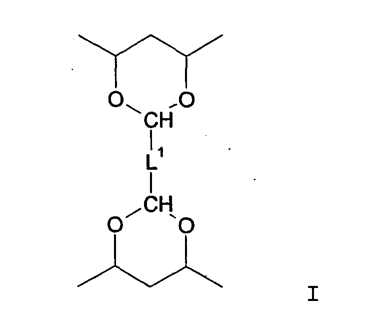 wherein L¹ is a residue of said cross-linking material.

Said cross-linking material preferably comprises a fourth polymeric material. Said fourth polymeric material preferably includes a repeat unit of formula 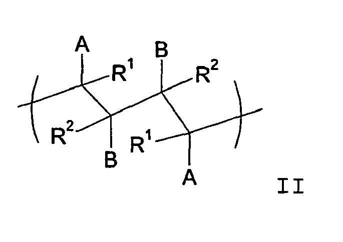 wherein A and B are the same or different, are selected from optionally-substituted aromatic and heteroaromatic groups and at least one comprises a relatively polar atom or group and R¹ and R² independently comprise relatively non-polar atoms or groups.

A and/or B could be multi-cyclic aromatic or heteroaromatic groups. Preferably, A and B are independently selected from optionally-substituted five or more preferably six-membered aromatic and heteroaromatic groups. Preferred heteroatoms of said heteroaromatic groups include nitrogen, oxygen and sulphur atoms of which oxygen and especially nitrogen, are preferred. Preferred heteroaromatic groups include only one heteroatom. Preferably, a or said heteroatom is positioned furthest away from the position of attachment of the heteroaromatic group to the polymer backbone. For example, where the heteroaromatic group comprises a six-membered ring, the heteroatom is preferably provided at the 4-position relative to the position of the bond of the ring with the polymeric backbone.

Preferably, A and B represent different groups. Preferably, one of A or B represents an optionally-substituted aromatic group and the other one represents an optionally-substituted heteroaromatic group. Preferably A represents an optionally-substituted aromatic group and B represents an optionally-substituted heteroaromatic group especially one including a nitrogen heteroatom such as a pyridinyl group.

Unless otherwise stated, optionally-substituted groups described herein, for example groups A and B, may be substituted by halogen atoms, and optionally substituted alkyl, acyl, acetal, hemiacetal, acetalalkyloxy, hemiacetalalkyloxy, nitro, cyano, alkoxy, hydroxy, amino, alkylamino, sulphinyl, alkylsulphinyl, sulphonyl, alkylsulphonyl, sulphonate, amido, alkylamido, alkylcarbonyl, alkoxycarbonyl, halocarbonyl and haloalkyl groups. Preferably, up to 3, more preferably up to 1 optional substituents may be provided on an optionally substituted group.

Unless otherwise stated, an alkyl group may have up to 10, preferably up to 6, more preferably up to 4 carbon atoms, with methyl and ethyl groups being especially preferred.

Preferably, A and B each represent polar atoms or group -that is, there is preferably some charge separation in groups A and B and/or groups A and B do not include carbon and hydrogen atoms only.

Preferably, at least one of A or B includes a functional group which can undergo a condensation reaction, for example on reaction with said third polymeric material. Preferably, A includes a said functional group which can undergo a condensation reaction.

Preferably, one of groups A and B includes an optional substituent which includes a carbonyl or acetal group with a formyl group being especially preferred. The other one of groups A and B may include an optional substituent which is an alkyl group, with an optionally substituted, preferably, unsubstituted, C₁₋₄ alkyl group, for example a methyl group, being especially preferred.

Preferably, A represents a group, for example an aromatic group, especially a phenyl group, substituted (preferably at the 4-position relative to polymeric backbone when A represents an optionally-substituted phenyl group) by a formyl group or a group of general formula 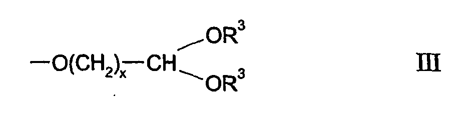 where x is an integer from 1 to 6 and each R³ is independently an alkyl or phenyl group or together form an alkalene group.

Preferably, B represents an optionally-substituted heteroaromatic group, especially a nitrogen-containing heteraromatic group, substituted on the heteroatom with a hydrogen atom or an alkyl or aralkyl group. More preferably, B represents a group of general formula 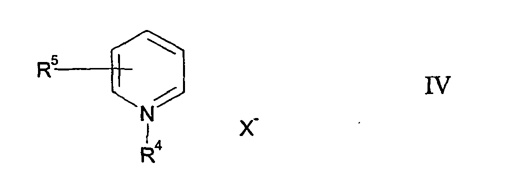 wherein R⁴ represents a hydrogen atom or an alkyl or aralkyl group, R⁵ represents a hydrogen atom or an alkyl group and X⁻ represents a strongly acidic ion.

Preferably, R¹ and R² are independently selected from a hydrogen atom or an optionally-substituted, preferably unsubstituted, alkyl group. Preferably, R¹ and R² represent the same atom or group. Preferably, R¹ and R² represent a hydrogen atom.

Preferred fourth polymeric materials may be prepared from any of the following monomers by the method described in WO98/12239 and the content of the aforementioned document is incorporated herein by reference:
α-(p-formylstyryl)-pyridinium, γ-(p-formylstyryl)-pyridinium, α-(m-formylstyryl)-pyridinium, N-methyl-α-(p-formylstyryl) -pyridinium, N-methyl-β-(p-formylstyryl)-pyridinium, N-methyl-α-(m-formylstyryl)-pyridinium, N-methyl-α-(o-formylstyryl)-pyridinium, N-ethyl-α-(p-formylstyryl)-pyridinium, N-(2-hydroxyethyl)-α-(p-formylstyryl)-pyridinium, N-(2-hydroxyethyl)-γ-(p-formylstyryl)-pyridinium, N-allyl-α-(p-formylstyryl)-pyridinium, N-methyl-y-(p-formylstyryl)-pyridinium, N-methyl-γ-(m-formylstyryl)-pyridinium, N-benzyl-α-(p-formylstyryl)-pyridinium, N-benzyl-γ-(p-formylstyryl)-pyridinium and N-carbamoylmethyl-γ-(p-formylstyryl)-pyridinium. These quaternary salts may be used in the form of hydrochlorides, hydrobromides, hydroiodides, perchlorates, tetrafluoroborates, methosulfates, phosphates, sulfates, methane-sulfonates and p-toluenesulfonates.

Also, the monomer compounds may be styrylpyridinium salts possessing an acetal group, including the following:

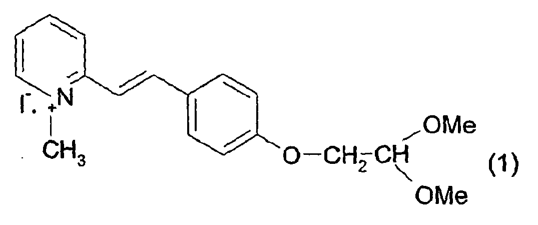

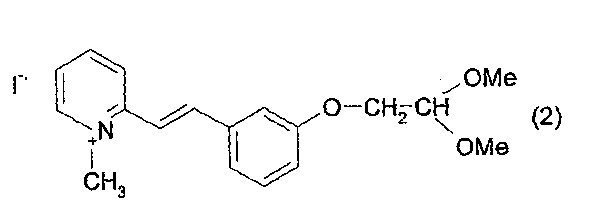

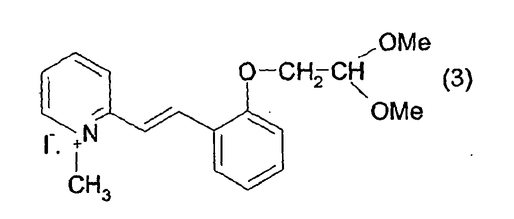

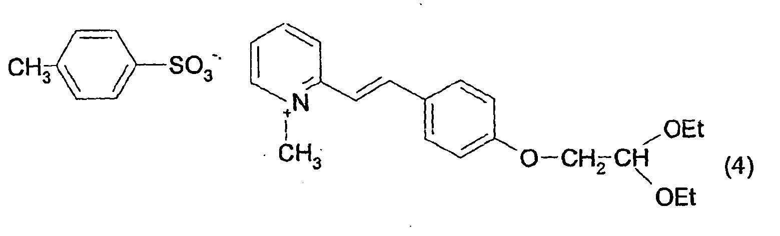

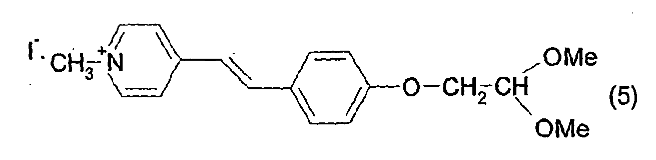

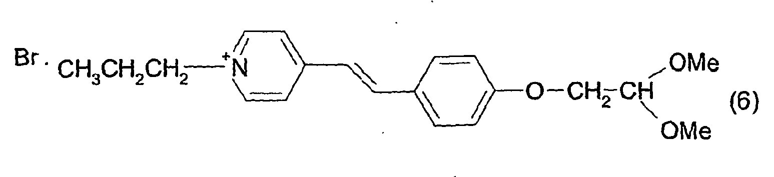

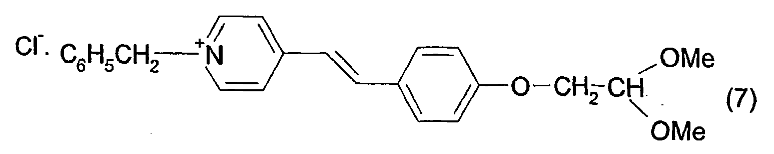

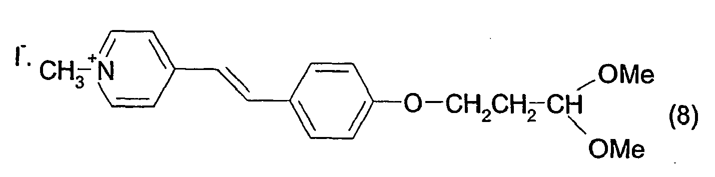

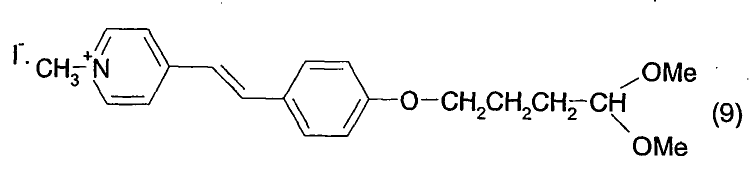

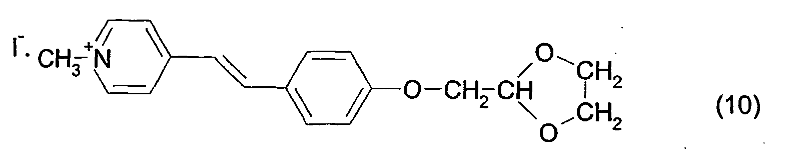

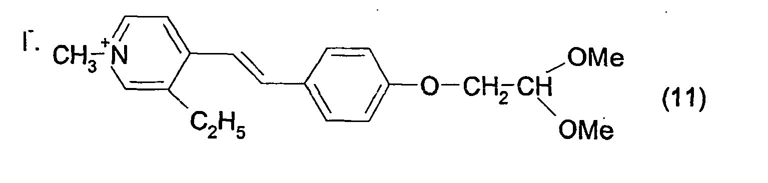

Thus, said fourth polymeric material is preferably prepared or preparable by providing a compound of general formula 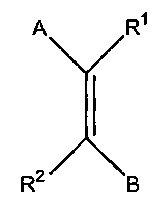 wherein A, B, R¹ and R² are as described above, in an aqueous solvent, (suitably so that molecules of said monomer aggregate) and causing the groups C=C in said compound to react with one another, (for example using UV radiation,) to form said fourth polymeric material.

Said fourth polymeric material may be of formula 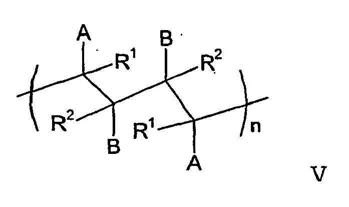 wherein A, B, R¹ and R² are as described above and n is an integer. Integer n is suitably 10 or less, preferably 8 or less, more preferably 6 or less, especially 5 or less. Integer n is suitably at least 1, preferably at least 2, more preferably at least 3. Preferably, formation of said first polymeric material from said third and fourth polymeric materials involves a condensation reaction. Preferably, formation of said first polymeric material involves an acid catalysed reaction. Preferably, said third and fourth polymeric materials include functional groups which are arranged to react, for example to undergo a condensation reaction, thereby to form said first polymeric material. Preferably, said third and fourth polymeric materials include functional groups which are arranged to react for example to undergo an acid catalysted reaction thereby to form said first polymeric material.

Said first polymeric material may be prepared by providing a mixture of said third polymeric material and said cross-linking material, especially said fourth polymeric material described, and causing the two materials to react. Preferably, said mixture includes at least 2wt%, more preferably at least 3wt% of said third polymeric material. When the molecular weight of the third polymeric material is relatively low (e.g. 50,000) the maximum amount of said third polymeric material in the mixture may be up to 40wt%. When the molecular weight of the third polymeric material is higher then the maximum amount may be less, for example up to 30wt%, or up to 20wt%. Said mixture may include at least 0.05wt%, preferably at least 0.1 wt% of said cross-linking means, especially said fourth polymeric material. The amount of said cross-linking means may be up to 3wt%.

Said third polymeric material and said cross-linking means are preferably provided in water. Said mixture may include at least 80wt%, suitably includes at least 85wt%, preferably includes at least 92wt%, more preferably includes at least 95wt%, especially includes at least 96wt% water. Said mixture may include other minor components, for example a catalyst, especially an acid, for catalysing the formation of said first polymeric material from said third polymeric material and said cross-linking means.

Said first polymeric material suitably includes a moiety of formula 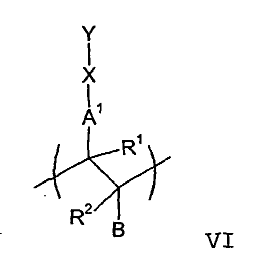 wherein R¹, R² and B are as described above, A¹ represents a residue of group A described above after the reaction involving said third and fourth polymeric materials, Y represents a residue of said third polymeric material after said reaction involving said third and fourth polymeric materials and X represents a linking atom or group extending between the residues of said third and fourth polymeric materials. In one preferred embodiment A¹ represents an optionally-substituted phenyl group, X represents a group 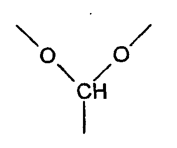 which is bonded via the oxygen atoms to a residue of said third polymeric material. For example, group X may be bonded to the polymer backbone of said third polymeric material.

The level of water may be reduced by any suitable means in step (b). Suitably, drying is undertaken at a temperature within the range 10°C to 60°C under atmospheric pressure or a lower pressure such as in a vacuum.

In step (c), the second polymeric containing a relatively low level of encapsulated water is treated with said derivatisation means. Derivatisation of said second polymeric material preferably includes a series of steps. In a first derivatisation step, said second polymeric material may be treated with a first derivatisation material which suitably reacts with said second polymeric material. A complex may be formed between the second polymeric material and said first derivatisation material or, preferably, reaction involves the formation of covalent bonds between the second polymeric material and the first derivatisation material. Preferably, the reaction of said second polymeric material and said first derivatisation material is carried out in the presence of less than 5wt%, preferably less than 1 wt% water. Preferably, the reaction is carried out in an organic solvent (e.g. acetone). Preferably, the reaction is carried out substantially in the absence of water. In this case, the reaction may predominantly take place on the surface of the second polymeric material with little penetration and reaction of reactants in microvoids in the polymeric material from which microvoids encapsulated water has been removed in step (b) of the method.

Said first derivatisation material may have any feature of the chemical cross-linking materials referred to above. When the first derivatisation step is carried out in an organic solvent and substantially in the absence of water, such a first derivatisation material should not (in the first derivatisation step) cross-link parts of the second polymeric material to any significant degree. Said first derivatisation material is preferably di-functional and preferably only one functional group of each molecule of the material reacts with the second polymeric material in said first derivatisation step. Preferably, said first derivatisation material includes at least one aldehyde group.

Said first derivatisation material may be selected from the monomers described above from which said fourth polymeric material may be prepared.

Derivatisation of the second polymeric material may include one or more derivatisation steps (including said first step described) arranged to introduce a linking moiety on said second polymeric material. The linking moiety is suitably arranged to link the second polymeric material to an active moiety. An active moiety may be selected to have desired properties and thereby provide a means whereby the desired properties may be associated with the derivatised material produced in the method. For example, the active material may be bio-compatible (and may therefore be arranged to increase the bio-compatibility of the first polymeric material) and/or it may be arranged to increase adhesion to cells. Preferred active materials include amino acid containing moieties, peptides and proteins. Alternatively, an active moiety may comprise a conducting polymer, organic semi-conductor or another material relevant to microelectronics interfacing technologies. In this case, the active moiety may be part of a sensor for monitoring cell chemistry or biology.

In some situations it may be possible to introduce an active moiety of the type described as part of said first step. However, said active moiety is suitably introduced in a step subsequent to said first step.

As described above, said first derivatisation step is suitably carried out substantially in the absence of water. One or more subsequent steps may also be carried out substantially in the absence of water. Alternatively, a subsequent derivatisation step may be carried out in the presence of water. Since it may be preferred that reactants in such a subsequent derivatisation step do not substantially penetrate into microvoids and react with functional groups other than those produced by derivatisation of said second polymeric material by said derivatisation means, when a derivatisation step is carried out in the presence of water, reactants and/or conditions are selected so the derivatisation step involves reaction with functional groups formed in an earlier derivatisation step. To achieve this, derivatisation may involve use of a material having a functional group which is unable to react with functional groups of the polymeric material other than those formed in an earlier derivatisation step or the speed of reaction of a selected material with groups formed in an earlier derivatisation step may be quicker than for other functional groups of the particular polymeric material.

Derivatisation of the second polymeric material preferably include a derivatisation step in which a compound having a amine group is reacted with the second polymeric material or a derivative thereof, for example a derivative which includes a linking group as described above.
Derivatisation with an amine group containing compound is preferably carried out in an aqueous solvent.

The method of the first aspect may involve increasing the level of encapsulated water after step (b). The level may be increased during treatment with derivatisation means in step (c) or subsequently. Advantageously, the strength of the first polymeric material after derivatisation and rehydration as described is comparable (in some cases it may be higher in some respects) to that of the first polymeric material selected in step (a).

The first polymeric material selected in step (a) may include predetermined microtopography and/or surface patterning and/or shape. Advantageously, in carrying out the method described, the microtopography, surface patterning and/or shape may be substantially retained after derivatisation of the material.

According to a second aspect of the invention, there is provided a method of making a polymeric material, the method comprising:
(a) selecting a fifth polymeric material which comprises:
   (i) a third polymeric material as described according to said first aspect cross-linked by a fourth polymeric as described according to said first aspect; or
   (ii) a polymeric material which includes a moiety of formula VI wherein R¹, R², B, A¹, X and Y are as described according to said first aspect; and
(b) treating said fifth polymeric material with derivatisation means for derivatising said fifth polymeric material, said derivatisation means being as described according to said first aspect.

Any feature of the first aspect may be applied to the second aspect mutatis mutandis.

The method of the first and second aspects may be used to introduce micropatterned surface chemistry on surfaces of a polymeric material derivatised in the methods. In this regard polymeric materials may be derivatised with a first moiety at predetermined positions on their surfaces with other positions on the surfaces being underivatised or dervatised with a different second moiety. In one embodiment, the first moiety may be arranged to render positions of the surface highly bio-compatible (e.g. adhesive of cells) whereas remaining areas of the surface may be less bio-compatible and/or may be arranged to block cell attachment.

Micropatterned surface chemistry may be produced by contacting the surface of the polymeric material at predetermined positions with a derivatisation means, for example a compound have an amine group as described above.

According to a third aspect of the invention, there is provided a derivatised polymeric material prepared or preparable in a method according to said first aspect or said second aspect.

According to a fourth aspect of the invention, there is provided a derivatised hydrogel. The hydrogel may be as described according to the first or second aspects.

Said hydrogel of the fourth aspect is preferably predominantly derivatised on surface regions thereof, in preference to macrovoids of the gel.

The derivatised polymeric material or hydrogel of the third and fourth aspects may include amide groups. Said amide groups are preferably part of a moiety pendent from a polymeric backbone. The amide groups preferably link a derivatisation means to the polymeric backbone. The amide groups preferably link an active material, for example an amino acid containing material, to a part of a moiety pendent from said polymeric backbone.

According to a fifth aspect of the invention, there is provided a method of preparing a material for a biological application, the method comprising forming microtopographical features in a surface of a first or second polymeric material according to said first aspect; or a fifth polymeric material according to said second aspect.

Said microtopographical features are preferably predetermined.

The polymeric material treated according to the third aspect may encapsulate water prior to, during and/or after formation of said microtopographical features. The method may include a step of reducing the level of encapsulated water after microtopographical features have been formed and optionally derivatising the polymeric material which includes said microtopographical features as described according to step (c) of the first aspect and step (b) of the second aspect. Advantageously, derivatisation may be restricted to predetermined positions on the surface of the polymeric material thereby to introduce micropatterned surface chemistry. The material produced may therefore incorporate both three dimensional surface patterning and patterned surface chemistry which may have benefits in certain biological applications such as dressings for wounds (or the like).

The formation of microtopographical features as described according to the fifth aspect may involve forming a template, for example a grating slide, incorporating a desired topography and contacting the template with a polymeric material in which the microtopographic features are to be formed. Preferably, contact with the polymeric material takes place prior to complete polymerisation of the polymeric material and contact continues as the material polymerises. When a polymeric material in which microtopographic features are to be formed is made from said third and fourth polymeric materials described above, the method may involve: mixing the third and fourth polymeric materials with any catalyst required, contacting the mixture with the template, and effecting reaction of the third and fourth polymeric materials; or a mixture comprising the third and fourth polymeric materials may be at least partially reacted prior to contact with the template. Thereafter, the reaction of the third and fourth polymeric materials may be completed.

Said microtopographical features may be predetermined by the provision of a template which is used to define the features in the method. The template may represent a negative of features produced in the first polymeric materials treated in the method of the fifth aspect.

Microtopographical features formed in accordance with said fifth aspect may comprise uniform shapes in the surface, for example channels or grooves which may have dimensions of less than 100µm.

According to a sixth aspect of the invention, there is provided a polymeric material, preferably for a biological application, the material comprising a polymeric material or hydrogel as described herein having microtopographical features.

The polymeric material or hydrogel may be as described according to the third or fourth aspects. Thus preferably, the invention provides a hydrogel having microtopographical features wherein said hydrogel is derivatised and preferably is derivated by an amino acid containing material.

According to a seventh aspect of the invention, there is provided a wound care product comprising a derivatised polymeric material or hydrogel according to any of the third, fourth, fifth or sixth aspects.

The wound care product may be a plaster or bandage (or the like) or, when usable internally, could be an implantable prosthesis (or the like). The term "wound" is intended to encompass defects caused by damage and/or disease.

According to an eight aspect of the invention, there is provided the use of a polymeric material or hydrogel according to the third, fourth, fifth or sixth aspects for the manufacture of a material for treatment of damaged and/or diseased tissues and/or wounds.

Any feature of any aspect of any invention or embodiment described herein may be combined with any feature of any aspect of any other invention or embodiment described herein.

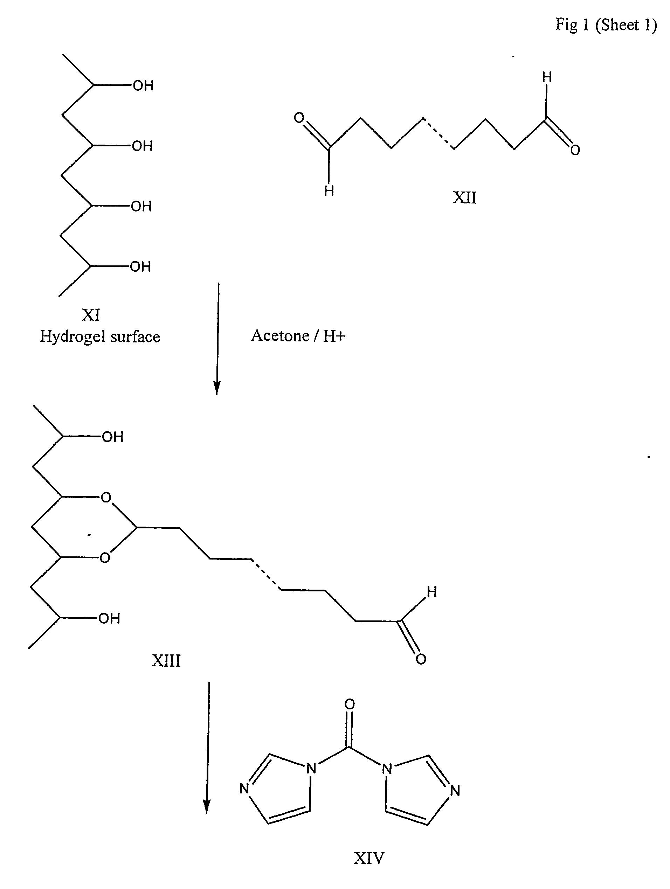

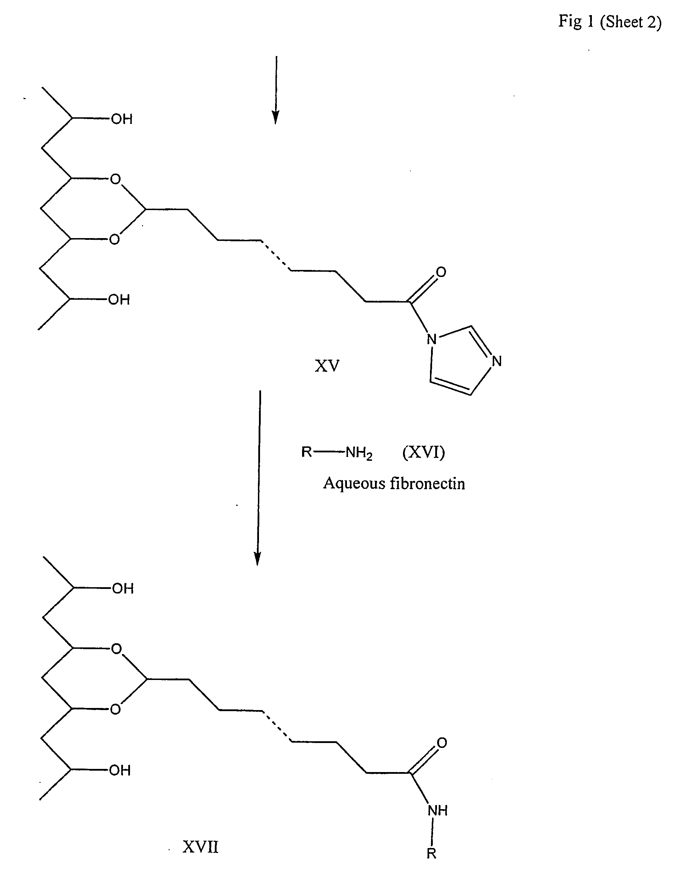

Specific embodiments of the invention will now be described, by way of example, with reference to Figure 1 which is a schematic representation of the derivatisation of a hydrogel with fibronectin.

The following examples describe how a hydrogel may be prepared and manipulated for use as a "smart" dressing. An objective in the preparation of a smart dressing is to recreate using microengineering techniques the microenvironmental conditions of the remodelling extracellular matrix of various types of wound which can then serve as an alternative highly permissive substratum for cell migration and cell division. To achieve oriented cell migration and division, it is important to present adherent cells with oriented surface shape in the form of microtopograhic guidance cues or micropatterned surface chemistry, in particular that required to initiate and accelerate cell motility. In general terms, in the embodiments which follow, means are provided whereby the microtopography of a surface of a hydrogel can be manipulated in a predetermined manner and, thereafter, the chemistry at the surface can be adjusted thereby to provide a material which can be used to achieve oriented cell migration and division.

### Example 1 - General method of preparing hydrogel

### Step (a) - Preparation of poly (1,4-di(4-(N-methylpyridinyl))_-2,3-di(4-(1-formylphenyl)butylidene

This was prepared as described in Example 1 of PCT/GB97/02529, the contents of which are incorporated herein by reference. In the method, an aqueous solution of greater than 1 wt% of 4-(4-formylphenylethenyl)-1-methylpyridinium methosulphonate (SbQ) is prepared by mixing the SbQ with water at ambient temperature. Under such conditions, the SbQ molecules form aggregates. The solution was then exposed to ultraviolet light. This results in a photochemical reaction between the carbon-carbon double bonds of adjacent 4-(4-formylphenylethenyl)-1-methylpyridinium methosulphate molecules (VIII) in the aggregate, producing a polymer, poly (1,4-di(4-(N-methylpyridinyl))-2,3-di(4-(1-formylphenyl)butylidene methosulphonate (IX), as shown in the reaction scheme below. It should be appreciated that the anions of compounds VIII and IX have been omitted in the interests of clarity.

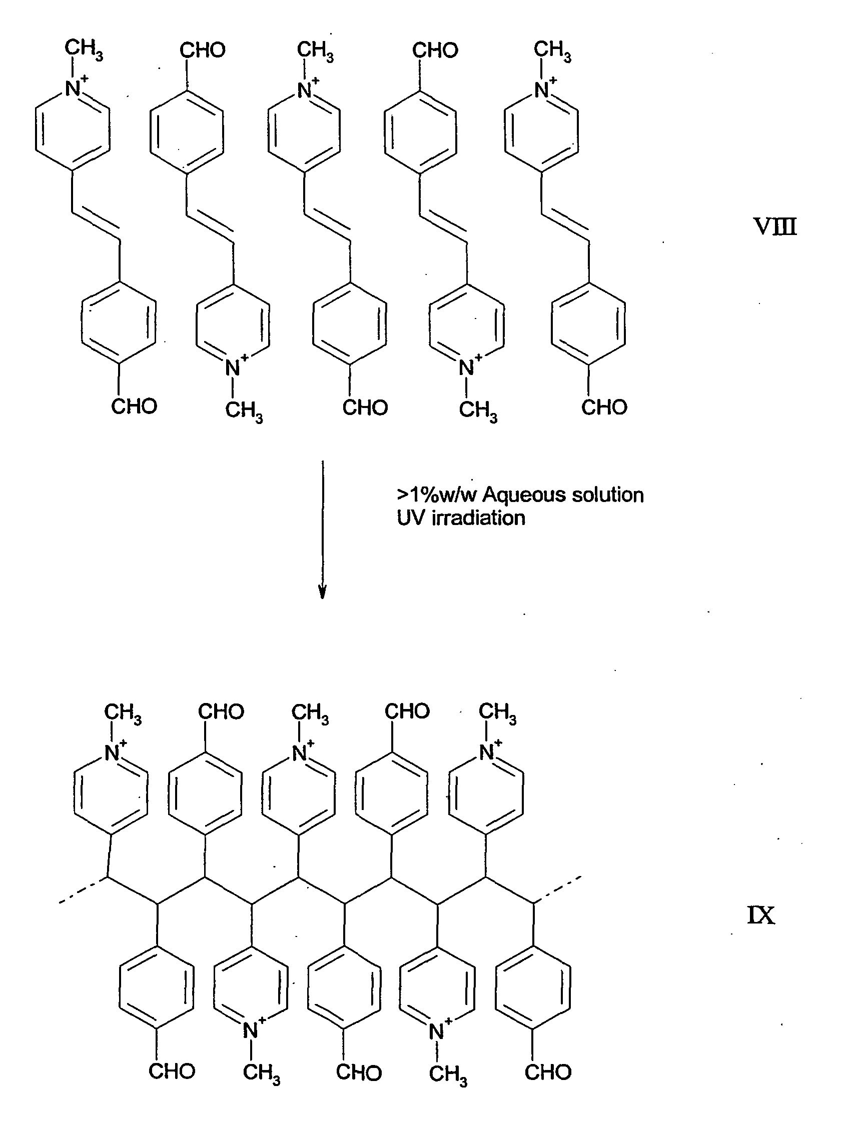

### Step (b)

A predetermined amount of 88% hydrolysed poly (vinylalcohol) of molecular weight 300,000 is dissolved in water by heating to 60°C for 6 hours. Then this is allowed to cool and a predetermined amount of the butylidene polymer of Step (a) is dissolved in the solution.

### Step (c)

An acid catalyst is added to the blend of Step (b) suitably to produce a pH of about 2. The mixture is then left to polymerise whereby the butylidene polymer of Step (a) cross-links the polyvinylalcohol according to the scheme below.

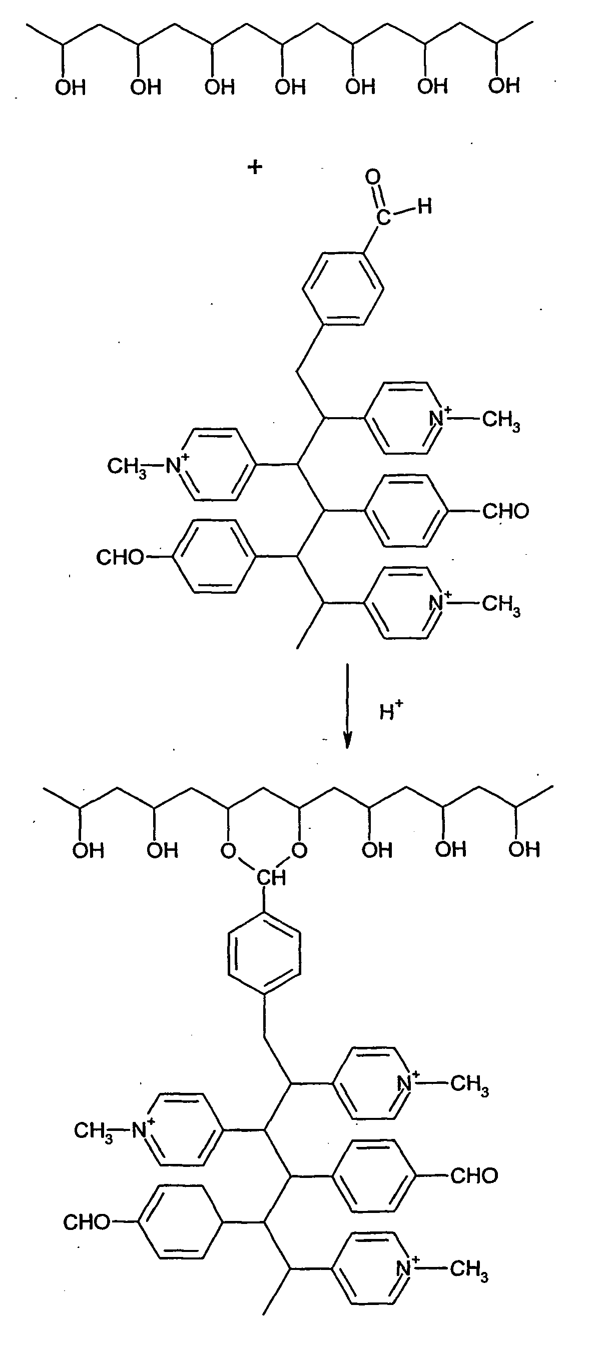

The concentration of the acid affects the speed of the cross-linking reaction. As cross-linking occurs a hydrogel is formed which can be treated and/or manipulated as described herein.

The hydrogel prepared may include 80-90wt% of entrapped water.

### Example 2 - General methodologies for preparing grating slides incorporating predetermined microtopography.

Oriented microtopographical cues are presented to cells as sets of grooves and ridges or channels embossed/cast into the surface of the gel using a master usually fabricated in fused silica or similar material. A desired microtopographic design is first drawn out as a lithographic resist mask either by photographic reduction or using a specific design package. A photoresist is patterned by exposure to UV through the lithographic mask followed by removal of exposed/non-exposed photoresist using a developing solution (either is possible depending on whether a positive or negative photoresist is used). This process may be used to fabricate features in the range of 10-looym in linear dimension. To produce microtopographic features smaller than this (e.g. of 50nm to 10µm) electron beam resists, usually of PMMA, are exposed using a beam writer whereby the design is directly written into the surface of the resist. For both types of device a grating slide incorporating predetermined microtopography is created by reactive ion etching the surface of a fused silica blank with gases such as C₂F₆ or equivalent using the patterned resist as an etch mask for sufficient time as is required to achieve a given etch depth.

Grating slides prepared may then be used to create microtopography on a hydrogel surface.

### Example 3 - Preparation of cast hydrogel incorporating microtopography

A selected grating slide made as described in Example 2, was cleaned with 10% decon (a detergent) solution in an ultrasonic bath for 40 minutes, rinsed copiously in-water, thoroughly dried and allowed to cool. The slide was then rinsed with 100% acetone and again allowed to dry. A petri dish was cleaned with heamosoal (a detergent), rinsed copiously in water, dried, and finally rinsed in 100% acetone, excess acetone being allowed to evaporate off. The slide was then placed in the petri dish, grating side up. A hydrogel formulation was prepared generally as described in Example 1 Steps(a)-(c), using 60g of a 10wt% solution of polyvinyl alcohol and 0.1 g of the polymer of Example 1, Step (c). The aqueous solution formed contains 10wt% polyvinyl alcohol and 0.5 wt% of the polymer of Example 1, step (c). The formulation in the presence of 1ml 20% HCl was mixed slowly, to reduce any air bubbles. The polymerising mixture was then poured into the petri dish and allowed to fully polymerise in a fume cupboard overnight. The dish was then placed into a vacuum oven overnight, at 50°C and -15mmHg pressure. After full polymerisation had occurred the hydrogel was peeled from the dish (and slide) and the grating formed in the hydrogel was cut out from the surrounding redundant hydrogel. It is found, on microscopic examination, that the hydrogel is able to provide a very good reproduction of the grating.

### Example 4 - Preparation of pulled hydrogel incorporating microtopography

A selected grating slide was prepared as described in Example 3 and, after drying, was placed on an inverted petri dish, grating side up. The hydrogel was prepared using 20g of a 10wt% solution of polyvinylalcohol and 0.1g of the polymer of Example 1, step (c). The aqueous solution formed contains 10wt% polyvinylalcohol and 0.5wt% of the polymer of Example 1, step (c). The aqueous solution was then mixed slowly with 0.2ml of 20% HCl, and then poured into a 5 cm petri dish to enable a hydrogel of a few mm thickness to be produced to enable it to be stretched. At a defined point in the polymerisation (when the hydrogel was not sticky enough to stick to a finger, but still quite 'wet') a corner of the hydrogel was taken in fingertips and stretched over the petri dish with grating. The hydrogel was left at room temperature for 1 hour and placed in an oven at 50°C for 45 minutes for full polymerisation to take place. After full polymerisation had occurred the hydrogel was carefully peeled from the dish (and slide) and the grating cut out from the redundant hydrogel.

Again, microscopic examination reveals that the hydrogel is able very precisely to reproduce the grating.

### Example 5 - General procedures for derivatising hydrogels

Any of the hydrogels described herein (whether incorporating microtopography or otherwise) may be derivatised to improve their bio-compatibility and/or to increase adhesion to cells, and such derivatised-hydrogels may be used in a wide range of applications. In preferred embodiments gels are dried prior to derivatisation. This may involve drying under vacuum at a temperature in the range 20 to 40°C for about 16 hours. Suitably, the gels are dried so that they contain less than 1wt% of water (measured e.g. by thermogravimetric analysis). It has been found that when dried gels are derivatised, the surface of the gel is predominantly derivatised (in preference to internal regions of the gel) from which a number of advantages may result. For example, bio-compatibility and/or the ability of the gels to adhere to cells will be concentrated at the surface of such derivatised gels, thereby restricting penetration into the body of the gel. Also, it is found that derivatisation may not significantly reduce the strength of the gels but may in some cases increase the strength. More particularly, derivatised dried gels may be rehydrated and the resultant hydrated derivatised gel may have comparable (or greater) strength compared to the non-derivatised version.

Hydrogels may be derivated using a range of methods. Firstly, a material, for example a bio-compatible material may be covalently bonded to the gel. In this regard, for the gel of Example 1 above, derivatisation may involve reactions involving hydroxyl, acetate or aldehyde groups on the gel. A linking group may be attached to the gel via such groups and then a desired bio-compatible material may be covalently attached to the linking group. Examples of suitable linking groups include the following:
- the polymer of Examples 1, Step (a). The polymer has aldehyde groups which may be reacted with hydroxy groups of the cross-linked polymer of Example 1, Step (c).
- the monomer (SbQ) described in Example 1, Step (a). Again this monomer has aldehyde groups which can be reacted with hydroxy groups.
- the monomers described on page 3 line 8 to line 39 of GB 2030575B and polymers prepared therefrom as described according to WO98/12239.

Secondly, a bio-compatible material may be associated with the gel by formation of a charge transfer complex. More particularly, a complex may involve interaction with an N⁺ moiety of a pyridinium moiety of the gel or a version of the gel derivatised with a pyridinum containing compound as described above. For example, a surfactant such as sodium lauryl sulphate may be attached to the gel using this methodology. In this case, the derivatised gel is less susceptible to subsequent swelling when hydrated and this may facilitate further reactions of the gel, for example of carbonyl groups thereof.

It will be appreciated that processes for derivatising the gel will be selected according to the nature of the bio-compatible material that it is desired to associate with the gel. In many embodiments, the bio-compatible material includes one or more amine groups (e.g. the material may be a protein or amino acid), in which case the material may be covalently bonded to the gel by an amide bond. Examples of materials that may be bonded to the gel in this way and which may improve the gels compatibility with cells include all known extracellular matrix components, cytokines, growth factors, hormones and other intra- or extracellular signalling molecules. A specific example is fibronectin.

### Example 6 - Derivatisation of hydrogel with fibronectin

Referring to figure 1, the hydrogel of Example 1, Step (c) (XI) having polyvinylalcohol moieties at its surface is treated with the butylidene polymer (XII) of Example 1, Step (b) in the presence of acetone and an acid-thereby to produce the condensation product XIII. Product XIII is then treated with carbonyl diimidazole (XIV) in acetone to produce XV which is treated with aqueous fibronectin (XVI) to produce the fibronectin derivatised hydrogel XVII.

In more detail, 2g of the polymer of Example 1, step (c) is added to 100ml of acetone and the solution stirred for 2 hours. This produces a saturated solution of the polymer of Example 1, step (c) in acetone (excess un-dissolved polymer is seen at the bottom of the flask). 0.5ml of concentrated hydrochloric acid is added to the solution. The dry hydrogel film is then immersed in the acidified polymer/acetone solution for at least 4 hours but not more than 16 hours. (By way of example, a dry hydrogel film of dimensions approx. 10cm diameter by 0.5mm thick requires 50 ml of the solution). The film is then washed with acetone several times and dried at 25°C under vacuum for approximately 4 hours.

100ml of a 1%w/v solution of carbonyl diimidazole in acetone is prepared and the hydrogel film prepared above is immersed in the solution for 4 hours. The film is then removed and again washed with acetone several times and dried under vacuum at 25°C for 4 hours.

Exposure of the above film to an aqueous solution of fibronectin results in covalent attachment of the fibronectin to the surface. The film is then washed with sterile distilled water.

### Example 7 - General procedure for producing micropatterned surface chemistry on the gel

Whilst the entire surface of the hydrogel may be derivatised as described in Examples 5 and 6, it is also possible to derivatise the gel in predetermined areas only thereby to produce desired surface chemistry in a predetermined pattern. A first method of achieving this involves the use of an elastomer stamp which has desired microtopography cast (or otherwise formed) into its surface. The elastomer is cured and soaked in a solution of an amine group containing compound (e.g. a protein such as fibronectin) for a time (about 20 minutes) for it to absorb sufficient of the protein for the application. The stamp is briefly exposed to a nitrogen stream to dry it. It is then placed contact side downwards on a hydrogel surface which has been derivatised with appropriate functionality so that it can react with the amine groups of the amine group containing compound (e.g. the stamp may be used to deliver fibronectin (XVI) to a gel derivatised as per compound XV of figure 1). After sufficient contact time (e.g. 1 minute) the stamp is withdrawn, leaving covalently bonded amine compound in predetermined positions on the gel. Thereafter, optionally, the non-derivatised areas may be treated with an alternative amine-group containing compound. The latter compound may be selected on the basis of the combinatorial effects of the two amine compounds to bind cells etc or it may act as a blocker for cell attachment. Examples of blockers are albumin and casein.

A second method of providing micropatterned surface chemistry involves the use of a stamp as described above except that in this case the stamp is planar. The stamp, loaded with amine containing compound, is moved by an operator to selected positions and contacted with the gel surface to define desired surface chemistry at the selected positions.

### Use of materials prepared

A gel, for example in the form of a film, incorporating microtopography and/or micropatterned surface chemistry may be formed in a suitable size to act as a dressing for a wound. The dressing is then applied engineered side down onto the wound itself. The wound may optionally be pre-treated by dermabrasion (or the like) to achieve appropriate viable cell-hydrogel contact. Studies on human skin cells and on model wounds have shown that the dressing can facilitate wound closure and/or the healing process.

The reader's attention is directed to all papers and documents which are filed concurrently with or previous to this specification in connection with this application and which are open to public inspection with this specification, and the contents of all such papers and documents are incorporated herein by reference.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings), may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s). The invention extends to any novel one, or any noel combination, of the features disclosed in this specification (including any accompanying claims, abstract and drawings), or to any novel one, or any novel combination, of the steps of any method or process so disclosed.

## Claims

1. A method of derivatising a polymeric material of a type which includes encapsulated water, the method comprising:
(a) selecting a first hydrated polymeric material which includes encapsulated water;
(b) reducing the level of encapsulated water in said first hydrated polymeric material to produce a second polymeric material;
(c) treating said second polymeric material with derivatisation means for derivatising said second polymeric material.

2. A method according to claim 1, wherein the difference between the wt% of water in said first polymeric material and that in said second polymeric material is at least 40wt% and said second polymeric material includes less than 10wt% of encapsulated water.

3. A method according to claim 1 or claim 2, wherein said first hydrated polymeric material comprises a third polymeric material which is cross-linked by a cross-linking means.

4. A method according to any preceding claim, wherein said first polymeric material is prepared by selecting a third polymeric material and treating it with a said cross-linking means, wherein said third polymeric material includes functional groups selected from hydroxyl, carboxylic acid, carboxylic acid derivatives and amine groups.

5. A method according to claim 3 or claim 4, wherein said third polymeric material is a polyvinyl polymer.

6. A method according to any of claims 3 to 5, wherein said third polymeric material is polyvinylalcohol.

7. A method according to any preceding claims, wherein said first polymeric material comprises cross-linked polyvinylalcohol.

8. A method according to any preceding claim, wherein said first polymeric material includes a moiety of formula I 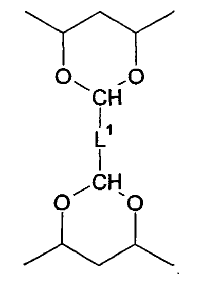 wherein L¹ is a residue of said cross-linking material.

9. A method according to claim 3 or claim 4, wherein said cross-linking means comprises a fourth polymeric material which includes a repeat unit of formula 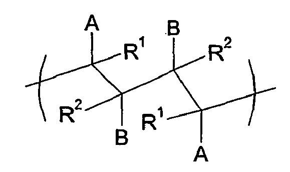 wherein A and B are the same or different, are selected from optionally-substituted aromatic and heteroaromatic groups and at least one comprises a relatively polar atom or group and R¹ and R² independently comprise relatively non-polar atoms or groups.

10. A method according to claim 9, wherein A and B are different, are selected from optionally-substituted aromatic and heteroaromatic groups and at least one of A or B comprises a relatively polar atom or group, R¹ and R² independently comprise relatively non-polar atoms or groups.

11. A method according to any preceding claim, wherein said first polymeric material includes a moiety of formula 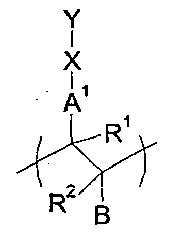 wherein R¹, R² and B are as described in claims 9 and 10, A¹ represents a residue of group A described in claims 9 and 10 after the reaction involving said third and fourth polymeric materials, Y represents a residue of said fourth polymeric material after said reaction involving said third and fourth polymeric materials and X represents a linking atom or group extending between the residues of said third and fourth polymeric materials.

12. A method according to any preceding claim, wherein, in step (b), drying is undertaken at a temperature in the range 10°C to 60°C.

13. A method according to any preceding claim, wherein, in step (c), said second polymeric material is derivatised in a first derivatisation step wherein said second polymeric material is treated with a first derivatisation material which reacts with said second polymeric material wherein said reaction is carried out in the presence of less than 5wt% water and is carried out in an organic solvent.

14. A method according to claim 13, wherein said first derivatisation material includes one or more carbonyl, carboxyl, hydroxyl, epoxy, halogen or amino functional groups.

15. A method according to claim 13 or claim 14, wherein said first derivatisation material is selected from compounds of general formula 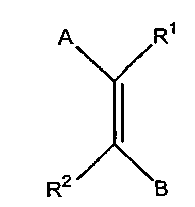 wherein A, B, R¹ and R² are as described in claims 9 and/or 10.

16. A method according to any preceding claim, wherein derivatisation of the second polymeric material includes one or more derivatisation steps arranged to introduce a linking moiety on said second polymeric material, wherein the linking moiety is arranged to link the second polymeric material to an active moiety.

17. A method according to claim 16, wherein the active moiety is biocompatible.

18. A method according to claim 16 or claim 17, wherein said active material is selected from amino acid containing moieties, peptides, proteins, conducting polymers, and organic semi-conductors or said active moiety may be part of a sensor for monitoring cell chemistry or biology.

19. A method according to any preceding claim, which involves increasing the level of encapsulated water at some stage after step (b).

20. A method of making a polymeric material, the method comprising:
(a) selecting a fifth polymeric material which comprises:
(i) a third polymeric material as described in claims 3 to 6 cross-linked by a fourth polymeric material as described in claims 9 or 10; or
(ii) a polymeric material which includes a moiety of formula VI as described in claim 11; and
(b) treating said fifth polymeric material with derivatisation means for derivatising said fifth polymeric material.

21. A derivatised polymeric material prepared or preparable in a method according to any of claims 1 to 19.

22. A method of preparing a material for a biological application, the method comprising forming microtopographical features in a surface of a first, second or fifth polymeric material according to any preceding claim.

23. A polymeric material selected from said first, second or fifth polymeric materials or a hydrogel according to any preceding claim having micro topographical features.

24. A wound care product comprising a derivatised polymeric material or hydrogel according to any preceding claim.

25. The use of a polymeric material or hydrogel according to any preceding claim for the manufacture of a material for treatment of damaged and/or diseased tissues and/or wounds.

## Patentansprüche

1. Verfahren zum Derivatisieren eines polymeren Materials eines Typs, welcher eingekapseltes Wasser einschließt, wobei das Verfahren umfasst:
(a) Auswählen eines ersten hydratisierten polymeren Materials, welches eingekapseltes Wasser einschließt;
(b) Verringern des Gehalts an eingekapseltem Wasser in dem ersten hydratisierten polymeren Material, um ein zweites polymeres Material zu erzeugen;
(c) Behandeln des zweiten polymeren Materials mit Derivatisierungsmitteln zum Derivatisieren des zweiten polymeren Materials.

2. Verfahren nach Anspruch 1, wobei der Unterschied zwischen den Gew.-% Wasser in dem ersten polymeren Material und denen in dem zweiten polymeren Material wenigstens 40 Gew.-% beträgt und das zweite polymere Material weniger als 10 Gew.-% eingekapseltes Wasser einschließt.

3. Verfahren nach Anspruch 1 oder Anspruch 2, wobei das erste hydratisierte polymere Material ein drittes polymeres Material umfasst, welches durch ein Vernetzungsmittel vernetzt ist.

4. Verfahren nach einem der vorangehenden Ansprüche, wobei das erste polymere Material durch Auswählen eines dritten polymeren Materials und Behandeln desselben mit einem besagten Vernetzungsmittel hergestellt wird, wobei das dritte polymere Material funktionelle Gruppen einschließt, die ausgewählt sind aus Hydroxyl, Carbonsäure, Carbonsäurederivaten und Amingruppen.

5. Verfahren nach Anspruch 3 oder Anspruch 4, wobei das dritte polymere Material ein Polyvinylpolymer ist.

6. Verfahren nach einem der Ansprüche 3 bis 5, wobei das dritte polymere Material Polyvinylalkohol ist.

7. Verfahren nach einem der vorangehenden Ansprüche, wobei das erste polymere Material vernetzten Polyvinylalkohol umfasst.

8. Verfahren nach einem der vorangehenden Ansprüche, wobei das erste polymere Material eine Einheit der Formel I einschließt 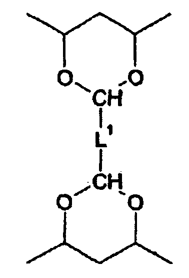 wobei L¹ ein Rest des vernetzenden Materials ist.

9. Verfahren nach Anspruch 3 oder Anspruch 4, wobei das Vernetzungsmittel ein viertes polymeres Material umfasst, welches eine Wiederholungseinheit der folgenden Formel einschließt 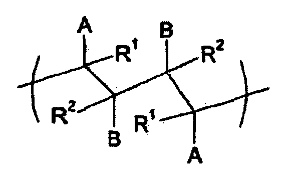 wobei A und B gleich oder voneinander verschieden sind, aus gegebenenfalls substituierten aromatischen und heteroaromatischen Gruppen ausgewählt sind und wenigstens eines ein relativ polares Atom oder eine relativ polare Gruppe umfasst und R¹ und R² unabhängig voneinander relativ unpolare Atome oder Gruppen umfassen.

10. Verfahren nach Anspruch 9, wobei A und B voneinander verschieden sind, aus gegebenenfalls substituierten aromatischen und heteroaromatischen Gruppen ausgewählt sind und wenigstens eines von A oder B ein relativ polares Atom oder eine relativ polare Gruppe umfasst, R¹ und R² unabhängig voneinander relativ unpolare Atome oder Gruppen umfassen.

11. Verfahren nach einem der vorangehenden Ansprüche, wobei das erste polymere Material eine Einheit der folgenden Formel einschließt 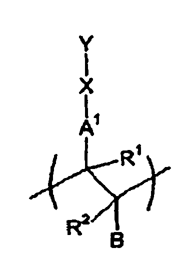 wobei R¹, R² und B wie in den Ansprüchen 9 und 10 beschrieben sind, A¹ einen Rest der Gruppe A, die in Ansprüchen 9 und 10 beschrieben ist, nach der Reaktion bedeutet, an der die dritten und vierten polymeren Materialien beteiligt sind, Y einen Rest des vierten polymeren Materials nach der Reaktion bedeutet, an der die dritten und vierten polymeren Materialien beteiligt sind, und X ein verbindendes Atom oder eine verbindende Gruppe bedeutet, das bzw. die sich zwischen den Resten der dritten und vierten polymeren Materialien erstreckt.

12. Verfahren nach einem der vorangehenden Ansprüche, wobei in Schritt (b) das Trocknen bei einer Temperatur im Bereich von 10°C bis 60 °C erfolgt.

13. Verfahren nach einem der vorangehenden Ansprüche, wobei in Schritt (c) das zweite polymere Material in einem ersten Derivatisierungsschritt derivatisiert wird, worin das zweite polymere Material mit einem ersten Derivatisierungsmaterial behandelt wird, welches mit dem zweiten polymeren Material reagiert, wobei die Reaktion in Gegenwart von weniger als 5 Gew.-% Wasser durchgeführt wird und in einem organischen Lösungsmittel durchgeführt wird.

14. Verfahren nach Anspruch 13, wobei das erste Derivatisierungsmaterial eine oder mehrere funktionelle Carbonyl-, Carboxyl-, Hydroxyl-, Epoxy-, Halogen- oder Aminogruppen einschließt.

15. Verfahren nach Anspruch 13 oder Anspruch 14, wobei das erste Derivatisierungsmaterial ausgewählt ist aus Verbindungen der allgemeinen Formel 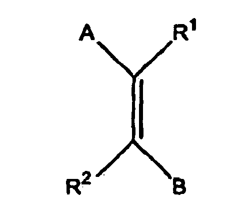 wobei A, B, R¹ und R² wie in den Ansprüchen 9 und/oder 10 beschrieben sind.

16. Verfahren nach einem der vorangehenden Ansprüche, wobei die Derivatisierung des zweiten polymeren Materials einen oder mehrere Derivatisierungsschritte einschließt, die so angeordnet sind, dass eine Verbindungseinheit an dem zweiten polymeren Material eingeführt wird, wobei die Verbindungseinheit so angeordnet ist, dass sie das zweite polymere Material mit einer aktiven Einheit verbindet.

17. Verfahren nach Anspruch 16, wobei die aktive Einheit biokompatibel ist.

18. Verfahren nach Anspruch 16 oder Anspruch 17, wobei das aktive Material ausgewählt ist aus Aminosäure enthaltenden Einheiten, Peptiden, Proteinen, leitenden Polymeren und organischen Halbleitern oder die aktive Einheit ein Teil eines Sensors zum Überwachen der Zellchemie oder -biologie sein kann.

19. Verfahren nach einem der vorangehenden Ansprüche, welches das Erhöhen des Gehalts an eingekapseltem Wasser in irgendeiner Stufe nach Schritt (b) umfasst.

20. Verfahren zum Herstellen eines polymeren Materials, wobei das Verfahren umfasst:
(a) Auswählen eines fünften polymeren Materials, welches umfasst:
(i) ein drittes polymeres Material, wie in den Ansprüchen 3 bis 6 beschrieben, vernetzt durch ein viertes polymeres Material, wie in den Ansprüchen 9 oder 10 beschrieben; oder
(ii) ein polymeres Material, welches eine Einheit der Formel VI, wie in Anspruch 11 beschrieben, einschließt; und
(b) Behandeln des fünften polymeren Materials mit Derivatisierungsmitteln zum Derivatisieren des fünften polymeren Materials.

21. Derivatisiertes polymeres Material, hergestellt oder herstellbar in einem Verfahren nach einem der Ansprüche 1 bis 19.

22. Verfahren zum Herstellen eines Materials für eine biologische Anwendung, wobei das Verfahren das Bilden von mikrotopografischen Merkmalen in einer Oberfläche eines ersten, zweiten oder fünften polymeren Materials gemäß einem der vorangehenden Ansprüche umfasst.

23. Polymeres Material, ausgewählt aus den ersten, zweiten oder fünften polymeren Materialien oder ein Hydrogel gemäß einem der vorangehenden Ansprüche mit mikrotopografischen Merkmalen.

24. Wundbehandlungsprodukt, umfassend ein derivatisiertes polymeres Material oder Hydrogel gemäß einem der vorangehenden Ansprüche.

25. Verwendung eines polymeren Materials oder Hydrogels gemäß einem der vorangehenden Ansprüche für die Herstellung eines Materials zur Behandlung von beschädigten und/oder erkrankten Geweben und/oder Wunden.

## Revendications

1. Procédé de dérivation d'un matériau polymère d'un type qui comprend de l'eau encapsulée, le procédé comprenant :
(a) la sélection d'un premier matériau polymère hydraté qui comprend de l'eau encapsulée ;
(b) la réduction de la teneur en eau encapsulée dans ledit premier matériau polymère hydraté pour produire un deuxième matériau polymère ;
(c) le traitement dudit deuxième matériau polymère avec des moyens de dérivation pour dériver ledit deuxième matériau polymère.

2. Procédé selon la revendication 1, dans lequel la différence entre le pourcentage en poids de l'eau dans ledit premier matériau polymère et celui dans ledit deuxième matériau polymère est d'au moins 40 % en poids et ledit deuxième matériau polymère comprend moins de 10 % en poids d'eau encapsulée.

3. Procédé selon la revendication 1 ou la revendication 2, dans lequel ledit premier matériau polymère hydraté comprend un troisième matériau polymère qui est réticulé par un moyen de réticulation.

4. Procédé selon l'une quelconque des revendications précédentes, dans lequel ledit premier matériau polymère est préparé par la sélection d'un troisième matériau polymère et par traitement de celui-ci avec ledit moyen de réticulation, dans lequel ledit troisième matériau polymère comprend des groupes fonctionnels choisis parmi les groupes hydroxyle, acide carboxylique, des dérivés d'acide carboxylique et amine.

5. Procédé selon la revendication 3 ou la revendication 4, dans lequel ledit troisième matériau polymère est un polymère de polyvinyle.

6. Procédé selon l'une quelconque des revendications 3 à 5, dans lequel ledit troisième matériau polymère est l'alcool polyvinylique.

7. Procédé selon l'une quelconque des revendications précédentes, dans lequel ledit premier matériau polymère comprend de l'alcool polyvinylique réticulé.

8. Procédé selon l'une quelconque des revendications précédentes, dans lequel ledit premier matériau polymère comprend un groupement de formule 1 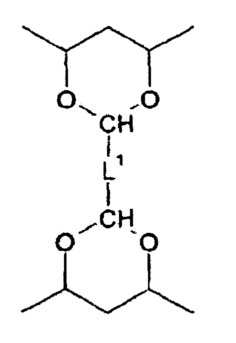 dans laquelle L¹ est un résidu dudit matériau de réticulation.

9. Procédé selon la revendication 3 ou la revendication 4, dans lequel ledit moyen de réticulation comprend un quatrième matériau polymère qui comprend un motif récurrent de formule 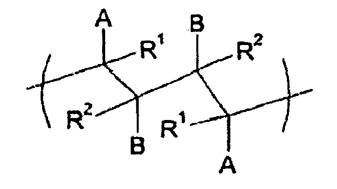 dans laquelle A et B sont identiques ou différents, sont choisis parmi les groupes aromatiques et hétéroaromatiques éventuellement substitués et l'un au moins comprend un atome ou un groupe relativement polaire et R¹ et R² comprennent indépendamment des atomes ou des groupes relativement non polaires.

10. Procédé selon la revendication 9, dans lequel A et B sont différents, sont choisis parmi les groupes aromatiques et hétéroaromatiques éventuellement substitués et l'un au moins parmi A et B comprend un atome ou un groupe relativement polaire et R¹ et R² comprennent indépendamment des atomes ou des groupes relativement non polaires.

11. Procédé selon l'une quelconque des revendications précédentes, dans lequel ledit premier matériau polymère comprend un groupement de formule 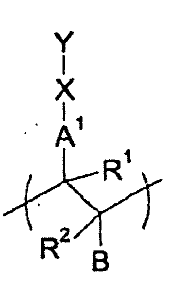 dans laquelle R¹, R² et B sont tels que décrits dans les revendications 9 et 10, A¹ représente un résidu du groupe A décrit dans les revendications 9 et 10 après une réaction mettant en jeu lesdits troisième et quatrième matériaux polymères, Y représente un résidu dudit quatrième matériau polymère après ladite réaction mettant en jeu lesdits troisième et quatrième matériaux polymères et X représente un atome ou un groupe de liaison s'étendant entre les résidus lesdits troisième et quatrième matériaux polymères.

12. Procédé selon l'une quelconque des revendications précédentes, dans lequel, dans l'étape (b), le séchage est réalisé à une température dans la plage allant de 10 °C à 60 °C.

13. Procédé selon l'une quelconque des revendications précédentes, dans lequel, dans l'étape (c), ledit deuxième matériau polymère est dérivatisé dans une première étape de dérivatisation dans laquelle ledit deuxième matériau polymère est traité avec un premier matériau de dérivatisation qui réagit avec ledit deuxième matériau polymère, où ladite réaction est réalisée en présence de moins de 5 % en poids d'eau et elle est réalisée dans un solvant organique.

14. Procédé selon la revendication 13, dans lequel ledit premier matériau de dérivatisation comprend un ou plusieurs groupes fonctionnels carbonyle, carboxyle, hydroxyle, époxy, halogéne ou amino.

15. Procédé selon la revendication 13 ou la revendication 14, dans lequel ledit premier matériau de dérivatisation est choisi parmi les composés de formule générale 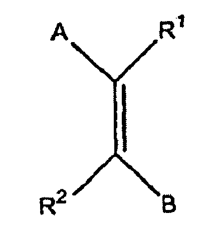 dans laquelle A, B, R¹ et R² sont tels que décrits dans les revendications 9 et/ou 10.

16. Procédé selon l'une quelconque des revendications précédentes, dans lequel la dérivatisation du deuxième matériau polymère comprend une ou plusieurs étapes de dérivatisation destinées à introduire un groupement de liaison sur ledit deuxième matériau polymère, où le groupement de liaison est destiné à lier le deuxième matériau polymère à un groupement actif.

17. Procédé selon la revendication 16, dans lequel le groupement actif est biocompatible.

18. Procédé selon la revendication 16 ou la revendication 17, dans lequel ledit matériau actif est choisi parmi les groupements contenant un acide aminé, les peptides, les protéines, les polymères conducteurs et les semi-conducteurs organiques ou ledit groupement actif peut faire partie d'un capteur destiné à suivre la chimie ou la biologie de cellules.

19. Procédé selon l'une quelconque des revendications précédentes, qui met en jeu l'augmentation de la teneur en eau encapsulée à un certain stade après l'étape (b).

20. Procédé de fabrication d'un matériau polymère, le procédé comprenant :
(a) la sélection d'un cinquième matériau polymère qui comprend :
(i) un troisième matériau polymère tel que décrit dans les revendications 3 à 6 réticulé par un quatrième matériau polymère tel que décrit dans la revendication 9 ou 10; ou
(ii) un matériau polymère qui comprend un groupement de formule VI tel que décrit dans la revendication 11 ; et
(b) le traitement dudit cinquième matériau polymère avec un moyen de dérivatisation pour dérivatiser ledit cinquième matériau polymère.

21. Matériau polymère dérivé préparé ou susceptible d'être préparé par un procédé selon l'une quelconque des revendications 1 à 19.

22. Procédé de préparation d'un matériau pour une application biologique, le procédé comprenant la formation de caractéristiques micro-topographiques sur une surface d'un premier, d'un deuxième ou d'un cinquième matériau polymère selon l'une quelconque des revendications précédentes.

23. Matériau polymère choisi parmi lesdits premier, deuxième ou cinquième matériaux polymères ou un hydrogel selon l'une quelconque des revendications précédentes comprenant des caractéristiques micro-topographiques

24. Produit de soin de blessures comprenant un matériau polymère dérivé ou un hydrogel selon l'une quelconque des revendications précédentes.

25. Utilisation d'un matériau polymère ou d'un hydrogel selon l'une quelconque des revendications précédentes pour la fabrication d'un matériau pour le traitement de tissus et/ou de blessures endommagé(e)s et/ou malades.
